# EUROPEAN PATENT APPLICATION

(11) **EP 3 447 122 A1**
(43) Date of publication of application: **27.02.2019**
(21) Application number: 17187763.2
(22) Date of filing: 24.08.2017
(51) Int. Cl.: C12N 1/20, A61K 35/742, A23K 10/18, C12R 1/125

(54) **BACILLUS SUBTILIS STRAIN WITH PROBIOTIC ACTIVITY**

(71) Applicant: Evonik Degussa GmbH, 45128 Essen (DE)
(72) Inventor: Pelzer, Stefan, 33335 Gütersloh (DE); Petri, Daniel, 1210 Wien (AT); Giatsis,Christos, 33602 Bielefeld (DE); Molck, Stella, 33602 Bielefeld (DE); Kipker, Maike, 51061 Köln (DE); Kleinbölting, Jessica, 33619 Bielefeld (DE); Stannek-Göbel, Lorena, 30175 Hannover (DE); Doranalli, Kiran, 60598 Frankfurt (DE); Htoo, John Khun Kyaw, 63755 Alzenau (DE); Borgmeier, Claudia, 64625 Bensheim (DE); Herbold, Sandra, 648163 Mannheim (DE)

(57) **Abstract**

The current invention concerns a new *B. subtilis* strain with strong inhibition of swine and poultry related pathogens and its use as probiotic.

## Description

The current invention concerns a new *B. subtilis* strain with strong inhibition of swine and poultry related pathogens and its use as probiotic.

The use of *B. subtilis* strains as probiotic ingredient in the feed industry has been disclosed before in the state of the art. The function of probiotics (also called "direct-fed microbials" or "DFM") is to influence the gut microflora in a positive way by supporting the growth of beneficial bacteria and/or the suppression of the growth of pathogenic bacteria. Ideally, by using probiotics the use of antibiotic growth promotors (AGPs) becomes redundant. But besides that, it is desirable that the probiotic fulfills further functions like helping in the digestion of specific feed ingredients.

Thus in view of the state of the art, there is a need for probiotics which influence the gut microflora in a positive way and beyond that desirably fulfill at least one further function.

Surprisingly it was found that the bacteria according to the current invention exhibit many advantageous features. Besides their ability to inhibit growth of *C. perfringens, C. difficile, S. gallinaceus*, *S. suis, C. coli, C. jejuni* and *E. cecorum*, the main commercially relevant pathogens of swine and poultry, they in particular show a very high proliferation rate in presence of bile and help to digest cellulose in a very effective way.

*Bacillus subtilis* DSM 32592 has been identified by targeted screening of naturally occurring isolates. It has been deposited with the DSMZ (Leibniz Institute DSMZ-German Collection of Microorganisms and Cell Cultures, Inhoffenstraße 7B, 38124 Braunschweig, Germany) on August 16, 2017 under the provisions of the Budapest Treaty on the International Recognition of the Deposit of Microorganisms for the Purpose of Patent Procedure under the Accession Number as mentioned before in the name of Evonik Degussa GmbH.

Thus a first subject of the current invention is a *Bacillus subtilis* strain and/or a preparation of said *Bacillus subtilis* strain selected from the following group:
a) The *Bacillus subtilis* strain as deposited under DSM 32592 at the DSMZ;
b) a mutant of the *Bacillus subtilis* strain as deposited under DSM 32592 having all identifying characteristics of the strain DSM 32592, wherein said mutant preferably has a DNA sequence identity to the strain DSM 32592 of at least 95%, preferably at least 96, 97 or 98 %, more preferably at least 99 or 99.5 % and/or wherein that mutant preferably has a genomic DNA sequence identity to the strain DSM 32592 of at least 95 %, preferably at least 96, 97 or 98 %, more preferably at least 99, 99.5 or 99.8 %, in particular of 100 %;
c) a preparation of (a) or (b);
d) a preparation containing an effective mixture of metabolites as contained in (a), (b) or (c).

The *Bacillus subtilis* strain as deposited under DSM 32592 at the DSMZ exhibits the following characterizing sequences:
a) a 16S rDNA sequence with a sequence identity of at least 99 or 99.5 %, in particular 100 %, to the polynucleotide sequence according to SEQ ID NO: 1;
b) a yqfD sequence with a sequence identity of at least 99 or 99.5 %, in particular 100 %, to the polynucleotide sequence according to SEQ ID NO: 2;
c) a gyrB sequence with a sequence identity of at least 99 or 99.5 %, in particular 100 %, to the polynucleotide sequence according to SEQ ID NO: 3;
d) an rpoB sequence with a sequence identity of at least 99 or 99.5 %, in particular 100 %, to the polynucleotide sequence according to SEQ ID NO: 4;
e) a groEL sequence with a sequence identity of at least 99 or 99.5 %, in particular 100 %, to the polynucleotide sequence according to SEQ ID NO: 5.

Thus, a further subject of the current invention is a *Bacillus subtilis* strain, in particular a *B. subtilis* strain with the characteristics as mentioned before, or a preparation thereof, wherein the *B. subtilis* strain exhibits at least one, preferably all, of the following characteristics:
a) a 16S rDNA sequence with a sequence identity of at least 99.5 %, more preferably at least 99.8 or 99.9 %, above all 100 %, to the polynucleotide sequence according to SEQ ID NO: 1;
b) a yqfD sequence with a sequence identity of at least 99.5 %, more preferably at least 99.8 or 99.9 %, above all 100 %, to the polynucleotide sequence according to SEQ ID NO: 2;
c) a gyrB sequence with a sequence identity of at least 99.5 %, more preferably at least 99.8 or 99.9 %, above all 100 %, to the polynucleotide sequence according to SEQ ID NO: 3;
d) an rpoB sequence with a sequence identity of at least 99.5 %, more preferably at least 99.8 or 99.9 %, above all 100 %, to the polynucleotide sequence according to SEQ ID NO: 4;
e) a groEL sequence with a sequence identity of at least 99.5 %, more preferably at least 99.8 or 99.9 %, above all 100 %, to the polynucleotide sequence according to SEQ ID NO: 5.

Thus, a further subject of the current invention is a *Bacillus subtilis* strain or a preparation thereof, in particular a *B. subtilis* strain with the characteristics as mentioned before, exhibiting two, three or four, preferably all, of the following characteristics:
a) a 16S rDNA sequence with a sequence identity of at least 99 %, preferably at least 99.5 %, more preferably at least 99.8 or 99.9 %, above all 100 %, to the polynucleotide sequence according to SEQ ID NO: 1;
b) a yqfD sequence with a sequence identity of at least 99 %, preferably at least 99.5 %, more preferably at least 99.8 or 99.9 %, above all 100 %, to the polynucleotide sequence according to SEQ ID NO: 2;
c) a gyrB sequence with a sequence identity of at least 99 %, preferably at least 99.5 %, more preferably at least 99.8 or 99.9 %, above all 100 %, to the polynucleotide sequence according to SEQ ID NO: 3;
d) an rpoB sequence with a sequence identity of at least 99 %, preferably at least 99.5 %, more preferably at least 99.8 or 99.9 %, above all 100 %, to the polynucleotide sequence according to SEQ ID NO: 4;
e) a groEL sequence with a sequence identity of at least 99 %, preferably at least 99.5 %, more preferably at least 99.8 or 99.9 %, above all 100 %, to the polynucleotide sequence according to SEQ ID NO: 5.

Thus, a particular subject of the current invention is also a *Bacillus subtilis* strain, exhibiting the following characteristics:
a) a 16S rDNA sequence according to SEQ ID NO: 1;
b) a yqfD sequence according to SEQ ID NO: 2;
c) a gyrB sequence according to SEQ ID NO: 3.
Preferably, this *B. subtilis* strain exhibits the following further characteristics:
d) an rpoB sequence according to SEQ ID NO: 4;
e) a groEL sequence according to SEQ ID NO: 5.

The strains of the current invention are preferably characterized by at least one, more preferably by all, of the following further features:

They are preferably able to grow under anaerobic conditions. Further, they are preferably able to degrade water-insoluble cellulose as well as protein under such anaerobic conditions.

Further, the strains of the current invention are preferably further able to degrade water-insoluble cellulose as well as protein under aerobic conditions, in particular in the presence of 2 mM bile.

They preferably inhibit infectious bacteria very effectively, in particular at least one strain, preferably all strains, selected from *C. perfringens* BB-081_Cpe; *C. perfringens* BB_031_Cpe; *C. difficile* DSM 1296; *S. gallinaceus* DSM 15349; *S. suis* DSM 9682, *C. coli* DSM 4689, *C. jejuni* DSM 24129 and *E. cecorum* DSM 20683.

In particular they are preferably characterized by a pathogen clearance of at least 15 mm, more preferably at least 20 mm, in a well diffusion antagonism assay on LBKelly agar plates with respect to *C. perfringens* strains BB-081_Cpe and BB_031_Cpe, respectively.

The strains according to the invention are preferably further characterized by exhibiting at least one, preferably all, of the following enzymatic activities: cellulase activity; xylanase activity; protease activity; catalase activity; superoxide dismutase activity.

In particular, they are preferably characterized by a xylanase activity of at least 15 mU/mL, more preferably at least 20 mU/mL, in particular about 26 mU/mL, and/or by a protease activity of at least 12 mU/mL, more preferably at least 16 mU/mL, in particular about 19 mU/mL, and/or by a cellulase activity of at least 500 mU/mL, more preferably at least 700 mU/mL, above all about 790 mU/mL.

The strains of the current invention preferably furthermore produce lactate and they are preferably further able to degrade mycotoxins.

The *B. subtilis* strains according to the invention are preferably further characterized by being able to grow in presence of 2 mM bile, more preferably in presence of 4 mM bile, and/or in presence of 0.3 wt.-% porcine bile and/or in presence of 0.3 wt.-% chicken bile. In particular they are preferably characterized by being able to proliferate fast under such high bile concentrations.

In addition, the strains are preferably able to grow under high salt conditions, in particular in presence of 5 wt.-% of NaCl, for at least one day.

Further the strains of the current invention, i.e. a significant part, in particular at least 80 %, of a respective sample, preferably survive the high temperatures necessary for pelleting animal feed, in particular they preferably survive a temperature of 80°C, more preferably of 95 or 99°C, for at least 20 minutes.

Without wishing to be bound by any theory, it is thought that the *Bacillus subtilis* strains according to the current invention enhance animal health, in particular gut health, by a multifaceted mode of action, including the production of antibacterial metabolites with selective efficacy and the competition with pathogenic bacteria by better consuming the available nutrients, thereby suppressing effective establishment of pathogenic bacteria in the gut. Hereby the enzymes produced by *Bacillus subtilis* may help to establish a balanced gut microbiota by providing predigested nutrients.

It is a big advantage of probiotics in comparison to antibiotics, that they do not attack bacteria indiscriminately nor do they lead to antibiotic resistant strains of pathogenic bacteria. Normally they are able to selectively compete with pathogenic bacteria by production of antimicrobial substances with specific efficacy, and are ideally able to simultaneously enhance the growth and viability of beneficial gut microflora. Further, they are preferably able to stimulate a systemic immune response in the treated animals.

The mutant strains of DSM 32592 of the current invention are preferably spontaneous mutants. The term "spontaneous mutant" refers to mutants that arise from DSM 32592 without the intentional use of mutagens. Such spontaneous mutants may be obtained by classical methods, such as growing the *Bacillus subtilis* strain in the presence of UV light and/or by applying high temperature or protoplast formation and/or in the presence of a certain antibiotic to which the parent strain is susceptible and testing any resistant mutants for improved biological activity or improved ability to enhance one or more of the indicia of animal health, in particular gut health. Other methods for identifying spontaneous mutants are known to those of ordinary skill in the art. But besides these preferred spontaneous mutants all other kinds of mutants of DSM 32592, like mutants obtained by genetic engineering, are also comprised by the current invention.

One particular embodiment of the current invention are naturally non-occurring mutants, in particular spontaneous mutants as defined before, of the strain DSM 32592, characterized by the features as mentioned above.

In a preferred embodiment of the current invention, the strains and preparations of the present invention are preferably administered orally to animals or human beings.

Thus, a further subject of the current invention are compositions, such as feedstuffs, foodstuffs, drinking and rearing water as well as therapeutic compositions, containing a *B. subtilis* strain and/or a preparation of the current invention.

A further subject of the current invention is also the use of a *B. subtilis* strain and/or a preparation of the current invention as a probiotic ingredient (DFM) in feed or food products.

Preferred foodstuffs according to the invention are dairy products, in particular yoghurt, cheese, milk, butter and quark.

The cells of the strains of the current invention may be present, in particular in the compositions of the current invention, as spores (which are dormant), as vegetative cells (which are growing), as transition state cells (which are transitioning from growth phase to sporulation phase) or as a combination of at least two, in particular all of these types of cells. In a preferred embodiment, the composition of the current invention comprises mainly or only spores.

In addition or as alternative the cells of the strains may also be used in non-living, inactivated form, as also the non-living cells are expected to still have a probiotic effect. Ways to inactivate the cells are known to those skilled in the art.

The *Bacillus subtilis* strains of the current invention and compositions containing them, when administered to animals, preferably enhance the health of such animals and/or improve the general physical condition of such animals and/or improve the feed conversion rate of such animals and/or decrease the mortality rate of such animals and/or increase the survival rates of such animals and/or improve the weight gain of such animals and/or increase the productivity of such animals and/or increase the disease resistance of such animals and/or increase the immune response of such animals and/or establish or maintain a healthy gut microflora in such animals and/or reduce the pathogen shedding through the feces of such animals. In particular the strains and compositions of the current invention might be used to assist in re-establishing a healthy balance of the gut microflora after administration of antibiotics for therapeutic purposes.

A further subject of the current invention is therefore a method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the productivity of animals and/or of increasing the disease resistance of animals and/or of increasing the immune response of animals and/or of establishing or maintaining a healthy gut microflora in animals and/or of reducing the pathogen shedding through the feces of animals, wherein the strains and/or preparations of the current invention or the compositions of the current invention, which comprise such strain(s), are administered to animals.

A further subject of the current invention is therefore also the use of strains and/or preparations and/or compositions of the current invention for enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for increasing the immune response of animals and/or for establishing or maintaining a healthy gut microflora in animals and/or for reducing the pathogen shedding through the feces of animals, wherein the strains and/or preparations of the current invention or the compositions of the current invention, which comprise such strain(s), are administered to animals.

A further subject of the current invention are therefore also the strains and preparations of the current invention as mentioned before and the compositions of the current invention, containing those strains, for enhancing the health of animals and/or for improving the general physical condition of animals and/or for improving the feed conversion rate of animals and/or for decreasing the mortality rate of animals and/or for increasing the survival rate of animals and/or for improving the weight gain of animals and/or for increasing the productivity of animals and/or for increasing the disease resistance of animals and/or for increasing the immune response of animals and/or for establishing or maintaining a healthy gut microflora in animals and/or for reducing the pathogen shedding through the feces of animals.

"Increasing the productivity of animals" refers in particular to any of the following: production of more or higher quality eggs, milk or meat or increased production of weaned offspring.

The methods and uses of the strains, preparations and compositions of the current invention can be therapeutic or non-therapeutic. In a particularly preferred embodiment of the current invention, the methods and uses are non-pharmaceutic, in particular feeding applications.

As the untreated manure of animals due to pathogenic bacteria and other ingredients may have a detrimental environmental effect, in particular with respect to the animals themselves and/or with respect to human beings getting in contact with the manure, which can be avoided by either feeding the animals or directly treating the manure or the bedding of the animals with the strains, compositions or preparations of the current invention, therefore a further subject of the current invention is a method of controlling and/or avoiding detrimental environmental effects of manure or contaminated liquids, the method comprising the step of applying to manure, contaminated liquids, litter, a pit, or a manure pond at least one strain, one preparation and/or one composition according to the current invention. Preferably the composition is applied in liquid form, for example by spraying, or as a powder, for example by strewing.

As detrimental bacteria may have a negative influence on the consistency of litter and in particular may effect a rather fluid or highly fluid litter, which might lead to foot pad lesions of poultry and which can be avoided by feeding the animals with the strains, compositions or preparations of the current invention, therefore a further subject of the current invention is a method of controlling and/or improving the consistency of litter, in particular a method of ensuring a solid consistency of litter and/or a method of avoiding foot pad lesions, the method comprising the step of feeding animals, in particular poultry, with at least one strain, one preparation and/or one composition according to the current invention.

The strains and preparations according to the invention can also be used for improving the quality of water. A further subject of the current invention is therefore also a method of controlling and/or improving the quality of water or aqueous solutions, in particular of drinking water and/or rearing water, comprising the step of applying to water or an aqueous solution at least one strain and/or at least one preparation and/or at least one composition of the current invention.

Further, the strains and preparations according to the invention can also be used for treating microbial diseases of plants. A further subject of the current invention is therefore also a method of treating and/or preventing microbial diseases of plants, in particular of cultivated plants, comprising the step of applying to the plants at least one strain and/or at least one preparation and/or at least one composition of the current invention. The application may be carried out in liquid form, such as by spraying, or in solid form, in particular as a powder, preferably as a formulated powder.

By using the strains, preparations and compositions of the current invention preferably an improvement of at least one of the features as mentioned before is realized, wherein realization of the feature preferably means an improvement of at least 1 %, more preferably of at least 3 or at least 5 %, in comparison to an adequate negative control. As negative control averages known in the animal husbandry field may be used, but preferably as negative control animals which are subjected to the same treatment like the animals tested are used, but without administration of the strains and/or preparations of the current invention.

In particular, the strains, preparations and compositions of the current invention may be administered or fed to an animal in an amount effective to inhibit and/or decrease the growth of pathogenic bacteria in the animal gut. Such pathogenic bacteria include *Clostridia*, *Listeria*, *Salmonella*, *Enterococci*, *Staphylococci, Aeromonas*, *Streptococci, Campylobacter, Escherichia coli, Shigella, Haemophilus, Brachyspira* and *Vibrio.* Relatedly, the methods of the present invention may be used to decrease the amount of pathogenic bacteria, viruses and protozoans shed in animal feces. The methods of the present invention may also be used to maintain or increase the growth of beneficial bacteria, such as lactic acid bacteria, in the animal gut. By decreasing pathogenic bacteria and/or increasing or maintaining beneficial bacteria, the compositions of the present invention are able to maintain an overall healthy gut microflora.

Thus, a further subject of the current invention are also the strains, preparations and compositions of the current invention for inhibiting and/or decreasing the growth of pathogenic bacteria and/or for maintaining and/or increasing the growth of beneficial bacteria in an animal gut, wherein the pathogenic bacteria are preferably selected from *Clostridia*, in particular from *C. perfringens, C. difficile*, *C. novyi*, *C. septicum* and *C. colinum,* from *Listeria*, in particular from *L. monocytogenes, L. seeligeri* and *L. welshimeri,* from *Salmonella*, in particular *S. enterica* including the subspecies *enterica, arizonae, bongori* and in particular the serovars, *S. gallinarum*, *S. pullorum, S. typhimurium, S. enteritidis*, *S. cholerasuis, S. heidelberg* and *S. infantis,* from *Enterococci,* in particular *E. faecalis*, *E. faecium* and *E. cecorum*, from *Staphylococci,* in particular *S. aureus*, from *Aeromonas*, from *Streptococci,* in particular *S. suis* and *S. gallinaceus*, from *Campylobacter,* in particular *C. jejuni* and *C. coli,* from *Escherichia coli,* from *Haemophilus,* in particular *Haemophilus parasuis,* from *Brachyspira, in particular Brachyspira hyodysenteriae* and from *Vibrio*, in particular *V. parahemolyticus* and *V. harveyi,* and the beneficial bacteria are preferably selected from lactic acid bacteria, in particular from lactobacilli and bifidobacteria.

In a preferred embodiment of the invention the amount of at least one pathogenic bacterium, in particular the amount of *C*. *perfringens,* is reduced by at least 0.5 log, more preferably by at least 1 log, 2 log, or 3 log.

Thus, a further subject of the current invention are also the strains, preparations and compositions of the current invention for inhibiting and/or decreasing the growth of pathogenic bacteria and/or for maintaining and/or increasing the growth of beneficial bacteria in an animal gut, wherein the pathogenic bacteria are preferably selected from *Clostridia*, in particular from *C. perfringens, C. difficile*, *C. novyi*, *C. septicum* and *C. colinum,* from *Listeria*, in particular from *L. monocytogenes, L. seeligeri* and *L. welshimeri,* from *Salmonella*, in particular *S. enterica* including the subspecies *enterica, arizonae, bongori* and in particular the serovars, *S. gallinarum*, *S. pullorum, S. typhimurium, S. enteritidis*, *S. cholerasuis, S. heidelberg* and *S. infantis*, from *Enterococci,* in particular *E. faecalis*, *E. faecium* and *E. cecorum*, from *Staphylococci,* in particular *S. aureus*, from *Aeromonas*, from *Streptococci,* in particular *S. suis* and *S. gallinaceus*, from *Campylobacter,* in particular *C. jejuni* and *C. coli,* from *Escherichia coli,* from *Haemophilus,* in particular *Haemophilus parasuis,* from *Brachyspira,* in particular *Brachyspira hyodysenteriae* and from *Vibrio*, in particular *V. parahemolyticus* and *V. harveyi,* and the beneficial bacteria are preferably selected from lactic acid bacteria, in particular from lactobacilli and bifidobacteria.

The occurrence and/or increased growth of the pathogenic bacteria does or can lead to the outbreak of certain diseases. For example the occurrence and/or increased growth of *Clostridium perfringens* can lead to the outbreak of gut diseases, in particular to the outbreak of necrotic enteritis in swine and poultry. The occurrence and/or increased growth of *C. perfringens* can also lead to the outbreak of further diseases like bacterial enteritis, gangrenous dermatitis and cholangiohepatitis. Even the mildest form of infection by *C. perfringens* can already be accompanied by diarrhea, which results in wet litter and by that may lead to secondary diseases like foot pad dermatitis. While *C. perfringens* type C generally is considered to be the primary cause of necrotic enteritis and necrohemorrhagic enteritis in piglets, type A has been linked to enteric disease in suckling and feeding pigs with mild necrotic enterocolitis and villous atrophy.

*Clostridium difficile* is an important emerging pathogen that causes diarrhea primarily in neonatal swine. Affected piglets may have dyspnea, abdominal distention, and scrotal edema.

*E. cecorum* is known to cause lameness, arthritis and osteomyelitis in broilers usually caused by an inflammation of a joint and/or bone tissue. Further E. cecorum can cause an inflammation of the pericardium.

*S. gallinaceus* can cause septicaemia in poultry. The gross lesions included splenomegaly, hepatomegaly, renomegaly and congestion. Multiple areas of necrosis and/ or infarction in the liver and spleen associated with valvular endocarditis were also observed.

*C. coli* is a foodborne bacterium, most people usually get infected by eating pig meat that contained the bacteria. It causes gastroenteritis and acute enterocolitis in humans, and also of acute diarrheal illnesses. Pigs are the main host, but it can also infect humans, avian species and a wide range of other animals.

Infection with *Campylobacter jejuni* is one of the most common causes of gastroenteritis worldwide [Acheson *et al.* 2001]. Infection results from the ingestion of contaminated food or water. *C. jejuni* is commonly associated with poultry, and it naturally colonises the digestive tract of many bird species [Colles et al. 2009] (20 to 100% of retail chickens are contaminated) as well as cattle especially calves. Some strains of *C. jejuni* have been reported to cause enteritis and death in newly hatched chicks and poults.

*S. suis* is an important pathogen in pigs and one of the most important causes of bacterial mortality in piglets after weaning causing septicemia, meningitis and many other infections.

Pathogens can cause further diseases like polyarthritis, fibrinous polyserositis, post-weaning enteric disorders like post-weaning diarrhea and edema disease and swine dysentery.

A further subject of the current invention is therefore also a therapeutic composition comprising the strains and/or compositions of the current invention as mentioned before.

A preferred subject in this context is therefore a therapeutic composition for treatment and/or prevention of necrotic enteritis and necrohemorrhagic enteritis, in particular sub-clinical necrotic enteritis and necrohemorrhagic enteritis, in animals, preferably swine or poultry, comprising the strains and/or compositions of the current invention as mentioned before.

Another preferred subject in this context is therefore a therapeutic composition for treatment and/or prevention of bacterial enteritis, gangrenous dermatitis, cholangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, in animals, preferably in swine or poultry, comprising the strains and/or compositions of the current invention as mentioned before.

A further subject of the current invention is therefore also the treatment and/or prevention of a disease, in particular of a gut disease, preferably of necrotic enteritis or necrohemorrhagic enteritis, in particular of sub-clinical necrotic enteritis or sub-clinical necrohemorrhagic enteritis, in swine or poultry, wherein a strain and/or composition and/or preparation of the current invention is administered to an animal in need thereof.

A further subject of the current invention is therefore also the treatment and/or prevention of a disease, preferably a disease of swine or poultry, selected from bacterial enteritis, gangrenous dermatitis, cholangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, wherein a strain and/or composition and/or preparation of the current invention is administered to an animal in need thereof.

The strains and/or preparations and/or compositions of the current invention can be administered to animals in feed and/or drinking water over multiple days throughout the animal's life or during particular stages or portions of the animal's life. For example, the strains and/or compositions can be administered only in a starter diet or only in a finisher diet of farm animals.

A particular subject of the current invention is also a method of enhancing the health of human beings and/or of improving the general physical condition of human beings and/or of increasing the disease resistance of human beings and/or of increasing the immune response of human beings and/or of establishing or maintaining a healthy gut microflora in human beings, wherein the strains and/or preparations of the current invention or the compositions of the current invention, which comprise such strain(s), are administered to human beings.

A further subject of the current invention is therefore also the use of strains and/or preparations and/or compositions of the current invention for enhancing the health of human beings and/or for improving the general physical condition of human beings and/or for increasing the disease resistance of human beings and/or for increasing the immune response of human beings and/or for establishing or maintaining a healthy gut microflora in human beings, wherein the strains and/or preparations of the current invention or the compositions of the current invention, which comprise such strain(s), are administered to human beings.

The compositions of the present invention, in particular the feed, food and pharmaceutical compositions as well as the drinking or rearing water, preferably comprise the strains of the current invention and are administered to animals at a rate of about 1x10³ to about 2x10¹² CFU/g feed or ml water, in particular in a rate of about 1x10³ or about 1x10⁴ or about 1x10⁵ or about 1x10⁶ or about 1x10⁷ or about 1x10⁸ or about 1x10⁹ or about 1x10¹⁰ or about 1x10¹¹ or about 1x10¹² CFU/g feed or ml water, preferably in an amount of about 1x10⁴ to about 1x10¹⁰ CFU/g feed or ml water, more preferably in an amount of 1x10⁴ to 1x10⁷ CFU/g feed or ml water.

Correspondingly, preferred amounts of the strains and/or preparations of the current invention in the feed, food and water compositions of the current invention range preferably from 0.1 wt.-% to 10 wt.-%, more preferably from 0.2 wt.-% to 5 wt.-%, in particular from 0.3 wt.-% to 3 wt.-%.

The methods of the present invention may be used for all kind of animals, in particular all kind of non-human and non-insect animals, more preferably all kind of vertebrates such as mammals, aquatic animals and birds.

Animals that may benefit from the current invention include but are not limited to farm animals, pets, exotic animals, zoo animals, aquatic animals, animals used for sports, recreation or work.

Pets are preferably selected from dogs, cats, domestic birds and domestic exotic animals.

Aquatic animals are preferably selected from finfish and crustaceans which are preferably intended for human nutrition. These include, in particular, carp, tilapia, catfish, tuna, salmon, trout, barramundi, bream, perch, cod, shrimps, lobster, crabs, prawns and crayfish. Preferred types of salmon in this context are the Atlantic salmon, red salmon, masu salmon, king salmon, keta salmon, coho salmon, Danube salmon, Pacific salmon and pink salmon.

Further preferred aquatic animals are farming fish which are subsequently processed to give fish meal or fish oil. In this connection, the fish are preferably herring, pollack, menhaden, anchovies, capelin or cod.

In a further preferred embodiment, the animals are farm animals, which are raised for consumption or as food-producers, such as poultry, swine and ruminants.

The poultry may be selected from productive or domestic poultry, but also from fancy poultry or wild fowl.

Preferred productive poultry in this context are chickens, turkeys, ducks and geese. The productive livestock in this context is preferably poultry optimized for producing young stock or poultry optimized for bearing meat.

Preferred fancy poultry or wild fowl are peacocks, pheasants, partridges, chukkars, guinea fowl, quails, capercaillies, grouse, pigeons and swans, with quails being especially preferred.

Further preferred poultry are ratites, in particular ostriches and emus, as well as parrots.

Ruminants according to the current invention are preferably selected from cattle, goat and sheep. In one embodiment, the compositions of this invention may be fed to preruminants to enhance their health and, in particular, to decrease the incidence of diarrhea in these animals. Preruminants are ruminants, including calves, ranging in age from birth to about twelve weeks.

The compositions of the current invention may comprise at least one carrier or typical feed ingredients or combinations thereof.

Suitable carriers are inert formulation ingredients added to improve recovery, efficacy, or physical properties and/or to aid in packaging and administration. Such carriers may be added individually or in combination. These carriers may be selected from anti-caking agents, anti-oxidation agents, bulking agents, and/or protectants. Examples of useful carriers include polysaccharides (in particular starches, maltodextrins, methylcelluloses, gums, chitosan and/or inulins), protein sources (in particular skim-milk powder and/or sweet-whey powder), peptides, sugars (in particular lactose, trehalose, sucrose and/or dextrose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), and silicates (in particular clays, in particular beolite clay, amorphous silica, fumed/precipitated silicas, zeolites, Fuller's earth, Baylith®, clintpolite, montmorillonite, diatomaceous earth, talc, bentonites, and/or silicate salts like aluminium, magnesium and/or calcium silicate). Suitable carriers for animal feed additives are set forth in the American Feed Control Officials, Inc.' s Official Publication, which publishes annually. See, for example Official Publication of American Feed Control Officials, Sharon Krebs, editor, 2006 edition, ISBN 1-878341-18-9. The carriers can be added after concentrating the fermentation broth and/or during and/or after drying. Preferred carriers according to the invention are selected from calcium carbonate, diatomaceous earth and vegetable oil.

A preferred embodiment of the current invention are concentrate compositions, in particular feed additive compositions, i.e. compositions suitable for preparing a feed composition, which comprise at least one strain of the current invention and at least one carrier as mentioned before, wherein the at least one strain is preferably comprised in an amount of 0.1 to 10 wt.-%, more preferably in an amount of 0.2 to 5 wt.-%, in particular in an amount of 0.3 to 3 wt.-%, above all in an amount of 0.4 to 2.2 wt.-%, and the at least one carrier is preferably comprised in an amount of at least 90 wt.-%, preferably in an amount of 90 to 99.9 wt.-%, more preferably in an amount of 95 to 99.8 wt.-%, in particular in an amount of 97 to 99.7 wt.-%, above all in an amount of 97.8 to 99.6 wt.-%, and wherein the carrier consists preferably substantially of limestone, in particular of limestone with smaller parts of diatomaceous earth and/or vegetable oil.

These preferred compositions of the current invention, which contain stabilized strains, can be used for the preparation of feed and pharmaceutical compositions as well as drinking and rearing water which preferably comprise the strains according to the invention in an amount as mentioned in the specification above. In a preferred embodiment 50 to 1000 grams of such a concentrate composition, in particular 50, 100, 250, 500 or 1000 grams of such a concentrate composition, are used per ton of feed, drinking or rearing water to provide compositions which can be used for feeding animals. These concentrate compositions preferably comprise at least one strain of the current invention in an amount of 1x10⁹ to 2x10¹¹ CFU, in particular 2x10⁹ to 1x10¹¹ CFU, per g of the concentrate composition.

Starting from these concentrate compositions, feed and food compositions can be prepared by mixing the concentrate compositions with typical feed or food ingredients, respectively.

Suitable typical animal feed ingredients which may be contained in the compositions according to the invention and/or used in the preparation of feed compositions starting from concentrate compositions according to the invention include one or more of the following: proteins, carbohydrates, fats, further probiotics, prebiotics, enzymes, vitamins, immune modulators, milk replacers, minerals, amino acids, coccidiostats, acid-based products and/or medicines, such as antibiotics.

Carbohydrates containing components which may be used according to the invention are for example forage, roughage, wheat meal, sunflower meal or soya meal, and mixtures thereof.

Proteins containing components which may be used according to the invention are for example soya protein, pea protein, wheat gluten or corn gluten, and mixtures thereof.
Fats containing components which may be used according to the invention are in particular oils, of both animal and plant origin, like vegetable oils, for example soya bean oil, rapeseed oil, sunflower seed oil, flaxseed oil or palm oil, fish oil, and mixtures thereof.

Proteins containing components which additionally contain fats which may be used according to the invention are for example fish meal, krill meal, bivalve meal, squid meal or shrimp shells, as well as combinations thereof.

Further probiotics (DFM) which may be used according to the invention in combination with the strains and preparations of the invention are preferably bacteria selected from the species *Bacillus subtilis, Bacillus licheniformis, Bacillus lentus*, *Bacillus pumilus, Bacillus laterosporus*, *Bacillus coagulans*, *Bacillus alevi*, *Bacillus cereus, Bacillus badius, Bacillus thurigiensis, Enterococcus faecium,* and *Pediococcus acidilactici.* Preferred examples are *Bacillus pumilus* DSM 32539 and *Bacillus subtilis* DSM 32540 (both deposited with the DSMZ on June 14, 2017 under the provisions of the Budapest Treaty on the International Recognition of the Deposit of Microorganisms for the Purpose of Patent Procedure) and derivatives thereof, *Bacillus licheniformis* DSM 32314 and *Bacillus subtilis* DSM 32315 (both deposited with the DSMZ on May 12, 2016 under the provisions of the Budapest Treaty on the International Recognition of the Deposit of Microorganisms for the Purpose of Patent Procedure) and derivatives thereof, *Bacillus subtilis* PB6 (as described in US Patent No. 7,247,299 and deposited as ATCC Accession No. PTA-6737), which is sold by Kemin under the trademark CLOSTAT®, *Bacillus subtilis* C-3102 (as described in US Patent No. 4,919,936 and deposited as FERM BP- 1096 with the Fermentation Research Institute, Agency of Industrial Science and Technology, in Japan), sold by Calpis as CALSPORIN®, *Bacillus subtilis* DSM 17299, as sold by Chr. Hansen under the trademark GalliPro®, *Bacillus licheniformis* DSM 17236, as sold by Chr. Hansen under the trademark GalliProTect®, a mixture of *Bacillus licheniformis* DSMZ 5749 and *Bacillus subtilis* DSMZ 5750 spores, as sold by Chr. Hansen under the trademark BioPlus®YC, *B. subtilis* DSM 29784, as sold by Adisseo/Novozymes under the trademark Alterion®, Bacillus subtilis, as sold by Chr. Hansen under the trademark PORCBOOST®, or *Bacillus coagulans* strains as described in US Patent No. 6,849,256. Other non-Bacillus probiotics, such as *Saccharomyces cerevisiae*, *Pichia pastoris, Aspergillus niger*, *Aspergillus oryzae,* or *Hansenula,* may also be used in compositions of the present invention. In particular in food compositions further probiotics which are known to be useful to the human health may be used such as lactic acid producing bacteria, in particular lactobacilli, or Bifidobacteria. If said further probiotics are not formulated as part of the compositions of the present invention, they may be administered together (either at the same time or at different times) with the compositions of the present invention.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used in feed compositions according to the invention and which may aid in the digestion of feed, are preferably selected from phytases (EC 3.1 .3.8 or 3.1.3.26), xylanases (EC 3.2.1.8), galactanases (EC 3.2.1 .89), galactosidases, in particular alpha-galactosidases (EC 3.2.1.22), proteases (EC 3.4), phospholipases, in particular phospholipases A1 (EC 3.1 .1.32), A2 (EC 3.1.1.4), C (EC 3.1.4.3), and D (EC 3.1.4.4), lysophospholipases (EC 3.1 .1.5), amylases, in particular alpha-amylases (EC 3.2.1.1); lysozymes (EC 3.2.1 .17), glucanases, in particular beta-glucanases (EC 3.2.1.4 or EC 3.2.1.6), glucoamylases, cellulases, pectinases, or any mixture thereof.

Examples of commercially available phytases include Bio-Feed™ Phytase (Novozymes), Ronozyme® P and HiPhos™ (DSM Nutritional Products), Natuphos™ (BASF), Finase® and Quantum® Blue (AB Enzymes), the Phyzyme® XP (Verenium/DuPont) and Axtra® PHY (DuPont). Other preferred phytases include those described in e.g. WO 98/28408, WO 00/43503, and WO 03/066847.

Examples of commercially available xylanases include Ronozyme® WX and G2 (DSM Nutritional Products), Econase® XT and Barley (AB Vista), Xylathin® (Verenium) and Axtra® XB (Xylanase/beta-glucanase, DuPont). Examples of commercially available proteases include Ronozyme® ProAct (DSM Nutritional Products).

Vitamins which may be used according to the invention are for example vitamin A, vitamin D3, vitamin E, vitamin K, e.g., vitamin K3, vitamin B12, biotin, choline, vitamin B1, vitamin B2, vitamin B6, niacin, folic acid and pantothenate, e.g. , Ca-D-pantothenate, or combinations thereof.

Immmune modulators which may be used are for example antibodies, cytokines, spray-dried plasma, interleukins, or interferons, or combinations thereof.

Minerals which may be used according to the invention are for example boron, cobalt, chloride, chromium, copper, fluoride, iodine, iron, manganese, molybdenum, selenium, zinc, calcium, magnesium, potassium, or sodium, or combinations thereof.

Amino acids which may be used according to the invention are for example lysine, alanine, threonine, methionine or tryptophan, or combinations thereof.

Thus, a further embodiment of the current invention is a method of preparing an animal feed composition comprising mixing at least one strain and/or at least one preparation and/or at least one concentrate composition of the current invention, in particular in an amount effective to enhance animal health, in particular gut health, with feed ingredients, such as proteins, lipids and/or carbohydrates, and optionally further beneficial substances, preferably as mentioned before, to provide a feeding product. This method may comprise for example also a pelleting step.

Standard pelleting processes known to those of skill in the art may be used, including extrusion processing of dry or semi-moist feeds. Preferred pelleting temperatures are between about 65° C and about 120° C.

The strains and compositions of the present invention can be obtained by culturing the strains of the current invention according to methods well known in the art, including by using the media and other methods as described for example in US 6,060,051, EP0287699 or US2014/0010792. Conventional large-scale microbial culture processes include submerged fermentation, solid state fermentation, or liquid surface culture. Towards the end of fermentation, as nutrients are depleted, the cells of the strains begin the transition from growth phase to sporulation phase, such that the final product of fermentation is largely spores, metabolites and residual fermentation medium. Sporulation is part of the natural life cycle of these strains and is generally initiated by the cell in response to nutrient limitation. Fermentation is configured to obtain high levels of colony forming units of the *Bacillus subtilis* cells and to promote sporulation. The bacterial cells, spores and metabolites in culture media resulting from fermentation may be used directly or concentrated by conventional industrial methods, such as centrifugation, tangential-flow filtration, depth filtration, and evaporation. The concentrated fermentation broth may be washed, for example via a diafiltration process, to remove residual fermentation broth and metabolites.

The fermentation broth or broth concentrate can be dried with or without the addition of carriers using conventional drying processes or methods such as spray drying, freeze drying, tray drying, fluidized-bed drying, drum drying, or evaporation. The resulting dry products may be further processed, such as by milling or granulation, to achieve a specific particle size or physical format. Carriers, as described above, may also be added post-drying.

Preparations of the strains of the current invention may be cell-free preparations or preparations containing cell debris or preparations containing a mixture of intact cells and cell debris.
Cell-free preparations of the strains of the current invention can be obtained for example by centrifugation and/or filtration of fermentation broth. Depending on the technique used, these cell-free preparations may not be completely devoid of cells, but may still comprise a smaller amount of cells. As the cells secret compounds like metabolites, enzymes and/or peptides into the surrounding medium, the supernatant of the cells comprises a mixture of such compounds, in particular metabolites, enzymes and/or peptides, as secreted by the cells. Thus, in a preferred embodiment of the invention, the preparation of the strains is a supernatant of the fermentation broth.

Compositions comprising cell debris of the strains may be obtained by rupturing the cells applying techniques as known to those of skill in the art, for example by mechanical means or by applying high pressure. Depending on the degree of force applied, a composition comprising only ruptured cells or a composition comprising a mixture of cell debris and intact cells is obtained. Homogenization of the cells may be realized for example by utilizing a French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer. Suitable alternatives are enzymatic and/or chemical treatment of the cells.

Cell-free preparations of the current invention comprise also preparations which are obtained by first rupturing the cells by applying techniques as mentioned before and subsequently removing the cell debris and the remaining intact cells. Removing of the cell debris and remaining intact cells can be carried out in particular by centrifugation and/or filtration.

The preparations of the strains of the current invention may comprise as active compounds at least one metabolite, preferably a mixture of metabolites, as further described below, and/or at least one enzyme selected from proteases, in particular subtilisin, xylanases and/or cellulases, and/or at least one peptide, and/or combinations thereof.

A preparation containing an effective mixture of metabolites as contained in the strains of the current invention and/or as contained in the cell preparations as mentioned before, can be obtained for example according to the methods set forth in US Patent No. 6,060,051. In particular the preparation can be obtained by precipitating the metabolites as contained in the preparations mentioned before by using organic solvents like ethyl acetate and subsequent redissolving of the precipitated metabolites in an appropriate solvent. The metabolites may subsequently be purified by size exclusion filtration that groups metabolites into different fractions based on molecular weight cut-off.

The preparation containing an effective mixture of metabolites of the current invention preferably comprises at least five, more preferably at least 6, 7, 8, 9, 10 or 12, in particular all metabolites of the strains of the invention. The content of metabolites of the strain DSM 32592 is depicted in Table 3.1. The metabolites possess preferably a molecular weight of between 500 and 4000 Dalton, more preferably of between 800 and 3500 Dalton.

Preferably according to the invention always an effective amount of the strains and/or preparations and/or compositions of the current invention is used in the embodiments of the current invention. The term "effective amount" refers to an amount which effects at least one beneficial effect to an animal and/or to the environment, in particular with respect to the features as already mentioned before, in comparison to an animal that has not been administered the strains and/or preparations and/or compositions of the current invention, but besides that has been administered the same diet (including feed and other compounds).

In case of therapeutic applications preferably a therapeutic amount of the strains and/or preparations and/or compositions of the current invention is used. The term "therapeutic amount" refers to an amount sufficient to ameliorate, reverse or prevent a disease state in an animal. Optimal dosage levels for various animals can easily be determined by those skilled in the art, by evaluating, among other things, the composition's ability to (i) inhibit or reduce pathogenic bacteria in the gut at various doses, (ii) increase or maintain levels of beneficial bacteria and /or (iii) enhance animal health, in particular gut health, at various doses.

### Working Examples

### Example 1. Strain characteristics relevant to survival in the gastrointestinal tract.

*Bacillus subtilis* strains were screened from various environmental samples in order to obtain a superior strain as animal direct-fed microbial / probiotic. As the strain is intended to reach its full potential in the intestine of the target animal, the strain was pre-screened to withstand various environmental and gut related conditions. Strain spores were generated (Nicholson and Setlow 1990), washed and incubated at 80 °C for 20 minutes (pasteurization), then titrated in logarithmic / 1 in 10 dilutions using veal infusion broth agar (VI, Difco™, no. 234420, Becton Dickinson GmbH, Heidelberg, Germany). The second highest dilution prior to no growth was stored at -80 °C and used as standardized starting point for all further assessments from spore state. To simulate gastric passage (Argenzio 2004a; Trampel and Duke 2004), survival of acid exposure was assessed based on Larsen *et al.* (2014). Growth of vegetative cells was furthermore assessed at low pH indicating growth under stomach/proventriculus and gizzard conditions, as well as in presence of up to 4 mM bile (B8631, CAS 8008-63-8, Sigma-Aldrich) at pH 7 in order to confirm strain growth at the proximal part of the small intestine right after clearance of the stomach or gizzard (Argenzio 2004b; Trampel and Duke 2004). Strain fitness in the anaerobe intestine (Argenzio 2004b; Trampel and Duke 2004) was assessed by inoculating standardized spore solutions under anaerobic conditions (AnaeroPak™, Thermo Fisher Scientific) in VI medium supplemented with 2.5 mM KNO₃ (Glaser et al. 1995). Furthermore was the anaerobe proteolytic and cellulytic activity of strains assessed on VI agar plates supplemented with 1% skim milk powder (70166, Sigma-Aldrich) or 0.1% water insoluble AZCL-HE cellulose (I-AZCEL, Megazyme International, Bray, Ireland). Furthermore the aerobe cellulytic activity of strains was assessed using VI agar supplemented with 0.1% water insoluble AZCL-HE cellulose (I-AZCEL, Megazyme International, Bray, Ireland). After 24 h under aerobe conditions, a blue coloration of agar indicated cellulase activity. Osmotic stress, as also found in the gut (Argenzio 2004b; Trampel and Duke 2004), was assessed by determining growth on VI agar with addition of 10 wt.-% NaCl (den Besten et al. 2009). Finally, spore heat stability was assessed to determine pelleting stability by exposing spores to 99 °C for 20 min (Palop et al. 1996) and subsequent inoculation on VI agar. *Bacillus subtilis* strain DSM 32592 survived simulated gastric passage, growth was observed starting at pH 6. Under aerobic conditions the strain DSM 32592 was able to degrade protein and water-insoluble cellulose in the presence of 2 mM bile. Strain DSM 32592 grew anaerobically and was able to degrade water-insoluble cellulose and protein under anaerobic conditions. Strain DSM 32592 was able to grow in presence of 2 and 4 mM bile, in presence of 0.3 wt.-% porcine bile and in presence of 0.3 wt.-% chicken bile as well as in presence of 5 wt.-% NaCl. Strain DSM 32592 reached an average spore count of 8.9 x 10⁸ CFU/mL, and spores of strain DSM 32592 were viable after exposure to 99°C for 20 min.

### References:

Argenzio, R. A. (2004a). Secretion of the Stomach and Accessory Glands, p. 405 - 418. In: Reece, W. O. (ed.), Duke's Physiology of Domestic Animals; Twelfth Edition, Chapter 25; Cornell University Press, Ithaca, New York, USA.
Argenzio, R. A. (2004b). Digestive and Absorptive Functions of the Intestines, p. 419 - 437. In: Reece, W. O. (ed.), Duke's Physiology of Domestic Animals; Twelfth Edition, Chapter 26; Cornell University Press, Ithaca, New York, USA.
Dawson, R. M. C.; Elliot, D. C.; Elliot, W. H.; Jones, K. M. (1986). Data for Biochemical Research; 3rd edition, Oxford Science Publishing, United Kingdom.
Den Besten HMW, Mols M, Moezelaar R, Zwietering MH, Abee T. (2009). Phenotypic and transcriptomic analyses of mildly and severely salt-stressed Bacillus cereus ATCC 14579 cells. Appl Environ Microbiol. 75:4111-9.
Glaser, P., A. Danchin, F. Kunst, P. Zuber, and M. M. Nakano. (1995). Identification and isolation of a gene required for nitrate assimilation and anaerobic growth of Bacillus subtilis. J. Bacteriol. 177:1112-1115
Nicholson W. L., Setlow P. Sporulation, germination and outgrowth. In: Harwood C R, Cutting S M, editors. Molecular biological methods for Bacillus. Chichester, England: John Wiley & Sons Ltd.; 1990. pp. 27-74.
Palop, A., Raso, J., Pagan, R., Condon, S. and Sala, F.J. (1996). Influence of pH on heat resistance of Bacillus licheniformis in buffer and homogenized foods. International Journal of Food Microbiology 29, 1-10.
Trampel, D. W. and Duke, G. E. (2004). Avian Digestion, p. 488 - 500. In: Reece, W. O. (ed.), Duke's Physiology of Domestic Animals; Twelfth Edition, Chapter 29; Cornell University Press, Ithaca, New York, USA.

### Example 2. Comparative strain performance relative to state of the art direct-fed microbial (DFM) / probiotic for animal nutrition - quantitative assessment of enzymatic activity.

Strains DSM 32592, DSM 17236 and DSM 17299 were compared evaluating the respective aerobe cellulytic, xylanolytic and proteolytic activity. Cellulase and xylanase activity were determined as described in Larsen *et al.* (2014). For proteolytic activity analysis, starter and main culture of strains were grown in VIB at 37°C as previously described. From main culture, 10 uL were added to 20 uL 0.5% Fluorescein Isothiocyanate Casein (FITC; C3777, Sigma-Aldrich) solution with 20 uL buffer consisting of 20mM sodium phospate (dibasic, anhydrous) with 150 mM sodium chloride (all components from Sigma-Aldrich), then incubated for 1 h at 37 °C. After addition of 150 uL of 10 % (v/v) trichlor acetic acid (Sigma-Aldrich) and another 30 min incubation at 37°C, samples were centrifuged at 19,000 rpm for 15 min, then 2 uL of supernatant transferred to 200 uL 500 mM TRIS HCl Solution (Trizma BaseTRIS, Sigma-Aldrich). Fluorescence of soluble peptides due to proteolytic release were determined (TECAN GENios Microplate Reader, Tecan Group Ltd., Männedorf, Switzerland) at excitation 494 nm, emission 518 nm. Analysis was performed in three independent runs, then averaged as milliunits per microliter solution. Results can be found in Table 2.1.

**Table 2.1. Xylanase, protease and cellulase activity of strains DSM 32592, DSM 17299 and DSM 17236.**

| Strain ID | Xylanase activity (mU/mL) | Protease activity (mU/mL) | Cellulase activity (mU/mL) |
|---|---|---|---|
| DSM 32592 | 26.4 ± 0.2 | 19.4 ± 2.4 | 790 ± 4 |
| DSM 17299 | 11.8 ± 1.5 | 9.8 ± 0.1 | 327 ± 20 |
| DSM 17236 | 7.9 ± 1.3 | 9.3 ± 0.3 | 62 ± 1 |

In direct comparison, strain DSM 32592 has a more than 2 fold increased xylanase activity, protease activity and cellulase activity compared to benchmark strains DSM 17236 and DSM 17299. In particular it has a more than 10 fold increased cellulose activity compared to benchmark strain DSM 17236.

### Reference:

Larsen, N., Thorsen, L., Kpikpi, E. N., Stuer-Lauridsen, B., Cantor, M. D., Nielsen, B., Brockmann, E., Derkx, E. M. F. and Jespersen, L. (2014). Characterization of Bacillus spp. strains for use as probiotic additives in pig feed. Applied microbiology and biotechnology, 98(3), 1105-1118.

### Example 3. Comparative strain performance relative to state of the art direct-fed microbial (DFM) / probiotic for animal nutrition - expression of metabolites and pathogen inhibition.

Strains DSM 32592 and wildtype strain DSM 10 were compared evaluating the respective number of metabolites expressed and pathogens inhibited in the respective media. For metabolite expression analysis, starter cultures were grown and tests performed as described in Scholz *et al.* (2011). From 10 mL Luria Bertami broth (LB, Thermo Fisher Scientific) culture grown for 24 h at 37°C and 160 rpm in 100 mL flask, 100 uL were transferred to main culture. Main culture was grown either in 10 mL LB containing 0.2 mL / L KellyT trace metal solution (LBKelly, Scholz *et al.* 2011), or 10 mL Trypticase Soy Broth (Oxoid, Thermo Fisher Scientific) with 0.6% yeast extract (Y1625, CAS 8013-1-2, Sigma-Aldrich; resulting broth abbreviated TSBYE), both for 24 h at 37°C at 160 rpm in 100 mL flask. Of the main culture, 4 mL were combined with 2 mL *n*-Butanol in 15 mL test tube, vortexed for 3 min, then sonicated for 15 min. After centrifugation for 1 min at 5000 rpm, organic phase was transferred, vacuum dried and analyzed using High-performance liquid chromatography - electrospray ionization mass spectrometry (HPLC-ESI-MS; Chen *et al.* 2006). Every sample was measured in two different modes, negative and positive mode, and mass spectra were acquired. Resulting peaks as similarly reported in Teo and Tan (2005) were converted to molecular mass in Da. Results for comparison can be found in Table 3.1.

**Table 3.1. (a) Comparison of metabolites expressed by strains DSM 32592 and wildtype strain DSM 10 in LB-Kelly and TSBYE, respectively (n/d means not detected)**

| Strain | 855 Da | 930 Da | 1008 Da | 1022 Da | 1026 Da | 1036 Da | 1040 Da |
|---|---|---|---|---|---|---|---|
| DSM 10 | n/d | yes | yes | yes | n/d | yes | n/d |
| DSM 32592 | yes | yes | yes | yes | yes | yes | yes |

**Table 3.1. (b) Comparison of metabolites expressed by strains DSM 32592 and wildtype strain DSM 10 in LB-Kelly and TSBYE, respectively (n/d means not detected)**

| Strain | 1050 Da | 1460 Da | 1462 Da | 1476 Da | 1504 Da | 1505 Da | 3401 Da |
|---|---|---|---|---|---|---|---|
| DSM 10 | yes | yes | yes | yes | yes | n/d | yes |
| DSM 32592 | yes | yes | yes | yes | yes | yes | yes |

In addition, pathogen inhibition via *Bacillus subtilis* secondary metabolite production, as part of metabolites from table 3.1. but not closer investigated, was assessed using well diffusion antagonism tests (Parente *et al.* 1995).
A well diffusion antagonisms test with 7 different pathogens, *Clostridium perfringens, Clostridium difficile* DSM 1296, *Enterococcus cecorum* DSM 20683, *Streptococcus gallinaceus* DSM 15349, *Streptococcus suis* ATCC 43765, *Campylobacter coli* ATCC 33559 (DSM 4689) and *Campylobacter jejuni* DSM 24129 was performed.

Two pathogenic *C. perfringens* candidates were tested being pathogenic *C. perfringens* field isolates from swine, obtained from RIPAC-LABOR GmbH, Potsdam-Golm, Germany. The *C. perfringens* type C-strains from Ripac describe as follows: Strains BB-081_Cpe and BB-031_Cpe were isolated from necrotic enteritis positive swine digestive tract. Strain BB-081_Cpe is cpb2 positive (Songer et al. 2005) and netB negative and Strain BB-031_Cpe tested positive for β2-toxin (Allaart et al. 2012).

*Clostridium difficile* is an important emerging pathogen that causes diarrhea primarily in neonatal swine (Songer et al. 2000). Affected piglets may have dyspnea, abdominal distention, and scrotal edema. Diarrhea may not be present in all pigs affected. DSM 1296 is a known type strain for C. *difficile* and produces cytotoxin.

*E. cecorum* is known to cause lameness, arthritis and osteomyelitis in broilers usually caused by an inflammation of a joint and/or bone tissue. Additional *E. cecorum* can cause an inflammation of the pericardium [Kense *et al.* 2011]. DSM 20683 was isolated from caecum of a chicken.

*S. gallinaceus* can cause septicaemia in poultry. The gross lesions included splenomegaly, hepatomegaly, renomegaly and congestion. Multiple areas of necrosis and/ or infarction in the liver and spleen associated with valvular endocarditis were also observed [Collins *et al.* 2002].

*S. suis* is an important pathogen in pigs and one of the most important causes of bacterial mortality in piglets after weaning causing septicemia, meningitis and many other infections [Goyette-Desjardins *et al.* 2014]. ATCC 43765 belongs to Serological group: R; serovar 2 and was isolated from pigs.

*C. coli* is a foodborne bacterium, most people usually get infected by eating pig meat that contained the bacteria. It causes gastroenteritis and acute enterocolitis in humans, and also of acute diarrheal illnesses [Fitzgerald *et al.* 2007]. Pigs are the main host, but it can also infect humans, avian species and a wide range of other animals. ATCC 33559 was isolated from pig feces.

Infection with *Campylobacter jejuni* is one of the most common causes of gastroenteritis worldwide [Acheson *et al.* 2001]. Infection results from the ingestion of contaminated food or water. *C. jejuni* is commonly associated with poultry, and it naturally colonises the digestive tract of many bird species [Colles et al. 2009] (20 to 100% of retail chickens are contaminated) as well as cattle especially calves. It is generally nonpathogenic in birds. Some strains of *C. jejuni* have been reported to cause enteritis and death in newly hatched chicks and poults. DSM 24129 is isolated from chickens.

*Bacillus* strains were grown in 10 mL TSBYE (30 g/l TSB + 6 g/l Yeast extract) or LB-Kelly (LB-Media supplemented with trace elements solution of DSMZ media 1032) for 16 h at 37°C and 200 rpm in 100 mL shaking flask. The pathogenic strains were grown under suitable conditions as liquid culture to an optical density of 595 nm of at least 1, then 100 µl were spread with sterile spatula on the surface of agar plates. For *S. gallinaceus* BHI agar plates, all other pathogens TSBYE agar plates are used. Three 9 mm diameter wells were cut into the dried plates. 1^{st} well was used as non-inoculated media control without culture, 2^{nd} well was inoculated with 100 uL not-inhibiting *Bacillus* strain (*B. cereus* var. *toyoi,* NCIMB 40112), the 3^{rd} well was inoculated with 100 uL of *Bacillus subtilis* DSM 32592 or DSM 17299 culture. After 24 h incubation under suitable conditions at 37°C, the zone of clearance in mm was determined measuring from the edge of the cut well to the border of the cleared lawn. Each colony was measured twice (horizontally, vertically), then averaged. The results can be found in the following tables.

**Table 3.2: Comparison of Bacillus subtilis DSM 32592, DSM 17236 and DSM 17299 inhibitory capacity on pathogenic Clostridium strains in well diffusion antagonism assays on LB Kelly medium, values in mm clearance of pathogen.**

| Pathogen → | *C. perfringens* | *C. perfringens* | *C. difficile* |
|---|---|---|---|
| Probiotic ↓ | BB-081_Cpe | BB_031_Cpe | DSM 1296 |
| DSM 32592 | 25.2 | 20.6 | 15.6 |
| DSM 17299 | 8 | 8 | n/d |
| DSM 17236 | 15.5 | 8 | 8 |

The data show that DSM 32592 is able to inhibit the growth of *C*. *perfringens* and *S. difficile* very effectively, in particular in comparison to DSM 17299.

**Table 3.3: Comparison of Bacillus subtilis DSM 32592 , DSM 17236 and DSM 17299 inhibitory capacity on pathogenic Staphylococcus, Streptococcus and Campylobacter strains in well diffusion antagonism assays on LB Kelly medium, values in mm clearance of pathogen.**

| Pathogen → | *S. gallinaceus* | *S. suis* | *C. coli* | *C. jejuni* |
|---|---|---|---|---|
| Probiotic ↓ | DSM 15349 | DSM 9682 | DSM 4689 | DSM 24129 |
| DSM 32592 | 19.0 | 22.6 | 22.3 | 15.1 |
| DSM 17299 | 8 | 13.5 | 8 | 8 |
| DSM 17236 | n/d | 20.7 | 8 | 8 |

The data show that DSM 32592 is able to inhibit the growth of *S. gallinaceus*, *S. suis, C. coli* and *C. jejuni* very effectively, in particular in comparison to DSM 17299.

**Table 3.4: Comparison of Bacillus subtilis DSM 32592, DSM 17236 and DSM 17299 inhibitory capacity on pathogenic Enterococcus cecorum in well diffusion antagonism assays on TSBYE medium, values in mm clearance of pathogen.**

| Pathogen → | *E. cecorum* |
|---|---|
| Probiotic ↓ | DSM 20683 |
| DSM 32592 | 18.7 |
| DSM 17299 | 8 |
| DSM 17236 | 13.7 |

The data show that DSM 32592 is able to inhibit the growth of *E. cecorum* very effectively, in particular in comparison to DSM 17299.

### References

Teo, A. Y.-L. and Tan, H.-M. (2005). Inhibition of Clostridium perfringens by a novel strain of Bacillus subtilis from the gastrointestinal tracts of healthy chickens. Appl. Environm. Microbiol., 71:4185-90.
Paul McMullin (2004) Chapter: Staphylococcus aureus subsp. aureus infections in chickens. Book: A pocket guide to poultry health and disease. Publisher: 5m Publishing.
Parente, E., Brienza, C., Moles, M., & Ricciardi, A. (1995). A comparison of methods for the measurement of bacteriocin activity. Journal of microbiological methods, 22(1), 95-108.
GA Contreras, JM Rodriguez (2011) Mastitis: Comparative Etiology and Epidemiology Journal of Mammary Gland Biology and Neoplasia, Volume 16, Issue 4, pp 339-356 (2016) U.S. Department of Health & Human Services (https://www.cdc.gov/foodsafety/diseases/staphylococcal.html)
Allaart, J. G., de Bruijn, N. D., van Asten, A. J., Fabri, T. H., and Gröne, A. (2012). NetB-producing and beta2-producing Clostridium perfringens associated with subclinical necrotic enteritis in laying hens in the Netherlands. Avian Pathol., 41:541-546
JG Songer, FA Uzal (2005) Clostridial Enteric Infections in Pigs. Volume: 17 issue: 6, page(s): 528-536 Journal of Veterinary Diagnostic Investigation
Songer JG, Post KW, Larson DJ, et al. (2000) Infection of neonatal swine with Clostridium difficile. Swine Health Prod. 2000;8(4):185-189
F M Colles, N D McCarthy, J C Howe, C L Devereux, A G Gosler, and M C J Maiden (2009) Dynamics of Campylobacter colonization of a natural host, Sturnus vulgaris (European Starling) Environ Microbiol. 2009 January; 11(1): 258-267.
Acheson, Allos (2001) Campylobacter jejuni Infections: Update on Emerging Issues and Trends Clinical Infectious Diseases, Volume 32, Issue 8, 15 April 2001, Pages 1201-1206
MJ Kense, WJM Landman (2011) Enterococcus cecorum infections in broiler breeders and their offspring: molecular epidemiology. Avian Pathology, Volume 40, Issue 6
MD Collins, RA Hutson, E Falsen, E Ingana, M Bisgaard (2002) Streptococcus gallinaceus sp. nov., from chickens. International Journal of Systematic and Evolutionary Microbiology, 52, 1161-1164
G Goyette-Desjardins, J-P Auger, J Xu, M Segura, M Gottschalk (2014) Streptococcus suis, an important pig pathogen and emerging zoonotic agent-an update on the worldwide distribution based on serotyping and sequence typing. Emerg Microbes Infect. 2014 Jun; 3(6):e45.
C Fitzgerald, I Nachamkin (2007). Campylobacter and Arcobacter. In P. R. Murray (Ed.), Manual of Clinical Microbiology (9th ed., pp. 933-946). Washington D.C.: ASM Press.

### Example 4. Qualitative assessment of antioxidant enzymatic activity.

The presence of reactive oxygen species during oxidative stress, which can be caused by stressful conditions as for example heat stress (Lin et al., 2006), can lead to a damage of DNA, proteins or lipids. Probiotics can support the host's oxidative defense system by increasing the antioxidant enzyme activities (Aluwong et al., 2013, Mishra et al., 2015). Therefore, the strain DSM 32592 was screened for antioxidant enzyme activities, in particular for superoxide dismutase and catalase activity.

For the evaluation of catalase activity of grown biofilms of the strain, the strain was grown in LB medium supplemented with glucose for 15 hrs at 37° C and 200 rpm in shaking flasks. The cultures were adjusted to an optical density OD₆₀₀ of 1.0 and 10 µl of the cultures were spotted onto TSBYE (30 g/l TSB + 6 g/l Yeast extract) or LB-Kelly (LB-Media supplemented with trace elements solution of DSMZ media 1032) agar plates, which were incubated at 37° C under aerobic conditions and at 37° C under 0.2 % oxygen for 15 hrs. 3 % H₂O₂ (hydrogen peroxide solution 3%, Sigma-Aldrich®) was dropped onto the colonies and catalase activity was analyzed. The read out parameter was the production of O₂ and H₂O from H₂O₂ resulting in the formation of foam on the colonies.

**Table 4.1. Assessment of catalase activity of colonies of Bacillus subtilis strain DSM 32592 grown as biofilm on LB-Kelly or TSBYE medium under aerobic conditions.**

| **Strain ID** | **TSBYE** | **LB-Kelly** |
|---|---|---|
| DSM 32592 | Yes | Yes |

*Bacillus subtilis* strain DSM 32592 displayed catalase activity when grown under aerobic conditions.

**Table 4.2. Assessment of catalase activity of colonies of Bacillus subtilis strain DSM 32592 grown as biofilm on LB-Kelly or TSBYE medium under 0.2 % oxygen.**

| **Strain ID** | **TSBYE** | **LB-Kelly** |
|---|---|---|
| DSM 32592 | Yes | Yes |

*Bacillus subtilis* strain DSM 32592 displayed catalase activity also when grown under 0.2% oxygen.

Catalase activity was analyzed in protein extracts obtained from planktonic cells grown under aerobic conditions as well. The strain DSM 32592 was grown for 15 hrs in LB medium containing glucose at 37° C and 200 rpm in shaking flasks. 8 ml of the cell cultures were harvested by centrifugation for 10 min at 4° C and 3000 rpm and the pellet was resuspended in PBS pH 7,3. Soluble cell extracts were obtained by using a ribolyser. The disrupted cells were centrifuged for 10 min at 4° C and 13000 rpm and the supernatant was used for further steps. The protein concentration of the protein extracts was determined by the method of Bradford with bovine serum albumin as a standard (Bradford, 1976). The concentration of the protein extracts was adjusted with PBS pH 7,3, the protein extracts were mixed with native sample loading buffer (2x, VWR) and native gel electrophoresis (10 % non-denaturing polyacrylamide gels, Biorad) was applied to separate the proteins at 4° C. Catalase activity in cell extrmolacts from planktonic cells was then detected by staining the gel in a staining solution of 1 % FeCl₃ and 1 % K₃Fe(CN)₆(Woodbury et al., 1971). Catalase activity can be seen as bright bands.

| **Strain ID** | **LB with glucose** |
|---|---|
| DSM 32592 | Yes |

*Bacillus subtilis* strain DSM 32592 displayed catalase activity also under these specific conditions as tested.

Superoxide dismutase activity was analyzed in protein extracts from cells grown under aerobic conditions as well. Cell extracts were obtained by the method described above and proteins were separated by performing native gel electrophoresis at 4° C. Superoxide dismutase activity was detected by staining the gel with a nitroblue tetrazolium staining method adapted from Beauchamp and Fridovich (1971).

| **Strain ID** | **LB with glucose** |
|---|---|
| DSM 32592 | Yes |

*Bacillus subtilis* strain DSM 32592 displayed superoxide dismutase activity under the conditions tested.

### References

Aluwong, T.; Kawu, M.; Raji, M.; Dzenda, T.; Govwang, F.; Sinkalu, V. and Ayo, J. (2013). Effect of Yeast Probiotic on Growth, Antioxidant Enzyme Activities and Malondialdehyde Concentration of Broiler Chickens. Antioxidants 2: 326-339 Beauchamp, C. and Fridovich, I. (1971). Superoxide dismutase: improved assays and an assay applicable to acrylamide gels. Anal. Biochem. 44: 276-287.
Bradford, M.M. (1976). A rapid and sensitive method for the quantitation of microgram quantities of proteins utilizing the principle of protein-dye binding. Anal. Biochem. 72: 248-254.
Lin, H.; Decuvpere, E. and Buyse, J. (2006). Acute heat stress incuces oxidative stress in broiler chickens. Comp. Biochem. Physiol. A. Mol. Integr. Physiol. 144: 11-17.
Mishra, V.; Shah, C.; Mokashe, N.; Chavan, R.; Yadav, H. and Prajapati, J. (2015). Probiotics as potential antioxidants: a systematic review. J. Agric. Food Chem 63: 3615-3626
Woodbury, W.; Spencer, A.K. and Stahmann, M.A. (1971). An improved procedure using ferricyanide for detecting catalase isozymes. Anal. Biochem. 44: 301-305.

### Example 5. Detection of lactate production

It has been shown in *in vitro* studies that inhibition of pathogen replication can be mediated by low-molecular-weight substances (Oelschläger 2010). Top of this list are short chain fatty acids, e.g. lactic acid (Oelschläger 2010). Possible explanation for the inhibition of pathogens can be decreasing the pH by production of lactic acid (Fuller 1992). Lactic acid bacteria, which are also used as probiotic in animal feed are known to produce lactic acid as major end-product during fermentation of cabohydrates (Halasz 2009). It is shown that *B. subtilis* strain DSM 32592 can produce lactate *in vitro.*

*B. subtilis* strain DSM 32592 and *Bacillus toyonensis -* a probiotic bacillus known to produce lactic acid - were compared evaluating the anaerobe lactate production. Lactate production was determined as follows: Precultures of the strains were grown stover night at 37 °C in 10 mL TSBYE (30 g/l TSB + 6 g/l Yeast extract) under aerobic conditions. Main culture was inoculated with an OD₆₀₀ of 0.2 in 10 ml TSBYE complemented with 25 g/l sucrose and 5 mM KNO₃ in 15 ml Falcon Tubes at 37 °C without shaking for 48 h. Lactate was measured in the supernatant with HPLC-UV.

| | % Lactate production |
|---|---|
| *Bacillus toyonensis* | 100.00 |
| DSM 32592 | 159.27 |

Strain *B. subtilis* DSM 32592 produces almost 50 % more lactate compared to probiotic strain of *Bacillus toyonensis.*

### References

A Halasz (2009) Book: Food quality and standards Volume III; EOLSS Publishers Co Ltd. Chapter: Lactic acid bacteria.
TA Oelschläger (2010) Mechanisms of probiotic actions - A review. International Journal of Medical Microbiology Volume 300, Issue 1, January 2010, Pages 57-62.
R Fuller 1992 Publisher: Springer science and business media Dordrecht Book: Probiotics: The scientific basis Chapter 9.8 Antimicrobial activity.

## Claims

1. A *Bacillus subtilis* strain or a preparation thereof selected from the following group:
a) The *B. subtilis* strain as deposited under DSM 32592 at the DSMZ;
b) a mutant of the *B. subtilis* strain as deposited under DSM 32592 having all identifying characteristics of the strain DSM 32592;
c) a preparation of (a) or (b);
d) a preparation containing an effective mixture of compounds as contained in (a), (b) or (c).

2. A *Bacillus subtilis* mutant of DSM 32592 according to claim 1 (b), wherein the mutant has a DNA sequence identity to the strain DSM 32592 of at least 95%.

3. A *Bacillus subtilis* strain or a preparation thereof, wherein the *B. subtilis* strain exhibits at least one, preferably all, of the following characteristics:
a) a 16S rDNA sequence with a sequence identity of at least 99.5 %, preferably at least 99.8 %, above all 100 %, to the polynucleotide sequence according to SEQ ID NO: 1;
b) a yqfD sequence with a sequence identity of at least 99.5 %, preferably at least 99.8 %, above all 100 %, to the polynucleotide sequence according to SEQ ID NO: 2;
c) a gyrB sequence with a sequence identity of at least 99.5 %, preferably at least 99.8 %, above all 100 %, to the polynucleotide sequence according to SEQ ID NO: 3;
d) an rpoB sequence with a sequence identity of at least 99.5 %, preferably at least 99.8 %, above all 100 %, to the polynucleotide sequence according to SEQ ID NO: 4;
e) a groEL sequence with a sequence identity of at least 99.5 %, preferably at least 99.8 %, above all 100 %, to the polynucleotide sequence according to SEQ ID NO: 5.

4. The *B. subtilis* strain according to any of the preceding claims, **characterized by** being able to grow anaerobically, in particular by being able to degrade water-insoluble cellulose and protein under anaerobic conditions.

5. The *B. subtilis* strain according to any of the preceding claims, **characterized by** being able to inhibit the growth of at least one, preferably all, of the strains selected from *C. perfringens* BB-081_Cpe; *C. perfringens* BB_031_Cpe; *C. difficile* DSM 1296; *S. gallinaceus* DSM 15349; *S. suis* DSM 9682; *C*. *coli* DSM 4689; *C. jejuni* DSM 24129 and *E. cecorum* DSM 20683.

6. The *B. subtilis* strain according to any of the preceding claims, **characterized by** at least one, preferably all, of the following enzymatic activities: cellulase activity; xylanase activity; protease activity; catalase activity; superoxide dismutase activity.

7. The *B. subtilis* strain according to any of the preceding claims, **characterized by** being able to grow in presence of 2 mM bile, preferably in presence of 4 mM bile, and/or in presence of 0.3 wt.-% porcine bile and/or in presence of 0.3 wt.-% chicken bile.

8. The use of *a B. subtilis* strain or a preparation thereof according to any of the preceding claims as a probiotic ingredient (DFM) in feed or food products.

9. A feed- or foodstuff composition containing a *B. subtilis* strain or a preparation thereof according to any one of claims 1 to 7 and at least one further feed or food ingredient, preferably selected from proteins, carbohydrates, fats, further probiotics, prebiotics, enzymes, vitamins, immune modulators, milk replacers, minerals, amino acids, coccidiostats, acid-based products, medicines, and combinations thereof.

10. A pharmaceutical composition containing a *B. subtilis* strain or a preparation thereof according to any one of claims 1 to 7 and a pharmaceutically acceptable carrier.

11. A composition according to claim 9 or 10 for improving the health status, in particular the gut health status, of an animal or a human being.

12. A method of feeding an animal, in particular swine or an avian, **characterized by** applying to the animal a *B. subtilis* strain or a preparation according to any of claims 1 to 7 and/or a feedstuff composition according to claim 9 and/or a pharmaceutical composition according to claim 10.

13. A method of improving the health status, in particular the gut health status, of an animal or a human being, comprising administering to the animal or human being a *B. subtilis* strain or a preparation according to any of claims 1 to 7 or a composition according to any of claims 9 to 11.

14. A method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the disease resistance of animals and/or of increasing the immune response of animals and/or of establishing or maintaining a healthy gut microflora in animals and/or of reducing the pathogen shedding through the feces of animals, wherein at least one strain and/or at least one preparation according to any of claims 1 to 7 or at least one composition according to any of claims 9 to 11 are administered to animals.

15. A method of controlling and/or avoiding detrimental environmental effects of manure or contaminated liquids, the method comprising the step of applying to manure, contaminated liquids, litter, a pit, or a manure pond at least one strain and/or at least one preparation according to any of claims 1 to 7 and/or at least one composition according to any of claims 9 to 11.

16. A method of controlling and/or improving the quality of water or aqueous solutions, in particular of drinking water or rearing water, the method comprising the step of applying to water or an aqueous solution at least one strain and/or at least one preparation according to any of claims 1 to 7 and/or at least one composition according to any of claims 9 to 11.

17. A method of treating and/or preventing a microbial disease of cultivated plants, comprising the step of applying to a cultivated plant at least one strain and/or at least one preparation according to any of claims 1 to 7 and/or at least one composition according to any of claims 9 to 11.


SEQUENCE LISTING

<110>  Evonik Degussa GmbH

<120>  Bacillus subtilis strain with probiotic activity

<130>  201700267

<160>  5

<170>  PatentIn version 3.5

<210>  1
<211>  1538
<212>  DNA
<213>  Bacillus subtilis

<400>  1
agagtttgat cctggctcag gacgaacgct ggcggcgtgc ctaatacatg caagtcgagc      60

ggacagatgg gagcttgctc cctgatgtta gcggcggacg ggtgagtaac acgtgggtaa     120

cctgcctgta agactgggat aactccggga aaccggggct aataccggat ggttgtttga     180

accgcatggt tcaaacataa aaggtggctt cggctaccac ttacagatgg acccgcggcg     240

cattagctag ttggtgaggt aacggctcac caaggcaacg atgcgtagcc gacctgagag     300

ggtgatcggc cacactggga ctgagacacg gcccagactc ctacgggagg cagcagtagg     360

gaatcttccg caatggacga aagtctgacg gagcaacgcc gcgtgagtga tgaaggtttt     420

cggatcgtaa agctctgttg ttagggaaga acaagtaccg ttcgaatagg gcggtacctt     480

gacggtacct aaccagaaag ccacggctaa ctacgtgcca gcagccgcgg taatacgtag     540

gtggcaagcg ttgtccggaa ttattgggcg taaagggctc gcaggcggtt tcttaagtct     600

gatgtgaaag cccccggctc aaccggggag ggtcattgga aactggggaa cttgagtgca     660

gaagaggaga gtggaattcc acgtgtagcg gtgaaatgcg tagagatgtg gaggaacacc     720

agtggcgaag gcgactctct ggtctgtaac tgacgctgag gagcgaaagc gtggggagcg     780

aacaggatta gataccctgg tagtccacgc cgtaaacgat gagtgctaag tgttaggggg     840

tttccgcccc ttagtgctgc agctaacgca ttaagcactc cgcctgggga gtacggtcgc     900

aagactgaaa ctcaaaggaa ttgacggggg cccgcacaag cggtggagca tgtggtttaa     960

ttcgaagcaa cgcgaagaac cttaccaggt cttgacatcc tctgacaatc ctagagatag    1020

gacgtcccct tcgggggcag agtgacaggt ggtgcatggt tgtcgtcagc tcgtgtcgtg    1080

agatgttggg ttaagtcccg caacgagcgc aacccttgat cttagttgcc agcattcagt    1140

tgggcactct aaggtgactg ccggtgacaa accggaggaa ggtggggatg acgtcaaatc    1200

atcatgcccc ttatgacctg ggctacacac gtgctacaat ggacagaaca aagggcagcg    1260

aaaccgcgag gttaagccaa tcccacaaat ctgttctcag ttcggatcgc agtctgcaac    1320

tcgactgcgt gaagctggaa tcgctagtaa tcgcggatca gcatgccgcg gtgaatacgt    1380

tcccgggcct tgtacacacc gcccgtcaca ccacgagagt ttgtaacacc cgaagtcggt 1440

gaggtaacct tttaggagcc agccgccgaa ggtgggacag atgattgggg tgaagtcgta 1500

acaaggtagc cgtatcggaa ggtgcggctg gatcacct 1538

<210> 2
<211> 1197
<212> DNA
<213> Bacillus subtilis

<400> 2
gtgaaaaata aatggctgtc ttttttttcg ggtaaggtcc agcttgaatt gacgggaaga 60

gggattgagc ggctccttaa tgaatgcaca agacagggga ttccggtctt tcatgtcaaa 120

aaaaagaaag aagccgtatc gttatatata cagcttcagg atgtacatgc ctttcggcgg 180

gtaagaagta aatttaaatg taaagcccga tttatcaatc ggaagggatt tcccttcctg 240

ttgctgaaat caaagctgaa tatagggttt acgatcggtt ttgcgatttt tttcattctt 300

ttgtttttgc tgtccaatat ggtgtggaaa attgatgtga caggcgctaa gcctgaaaca 360

gaacatcaaa tgaggcagca tcttaatgaa atcggcgtca aaaagggccg tctgcagttt 420

ttaatgatgt cgcccgaaaa aatacagaaa tcattaacca atggaataga caatatcact 480

tgggtcggag ttgatctgaa ggggacgacc attcatatga aagttgtgga gaaaaatgag 540

cccgaaaaag aaaaatatgt tagcccgcgc aatattgtcg ccaaaaagaa agcaaccatt 600

acgagaatgt ttgtgcaaaa aggacagccc atggccgcca tacacgatca tgttgaaaag 660

ggacagctgc ttgtttcggg actgatcggc agcgaagacc atcagcagga agtcgcctca 720

aaagcagaaa tttatggaga aacctggtat agatcagaag tgacagtccc gcttgaaaca 780

ttatttaacg tctatacggg caaagtaagg acaaagcaca agctttcttt tggttctttg 840

gcaatcccga tctgggggat gacgtttaaa aaagaggaat tgaagcatcc aaaaacagaa 900

caagaaaagc attcgcttca ttttctcgga tttaagctcc ctgtatccta tgtcaaagag 960

caaacgagag aaagtgaaga ggctttgcga aaatatacaa aagaagaagc agttcaagaa 1020

ggcattaaat tgggtaaaca ggatgtagag gataaaatag gcgaaaacgg cgaggtgaaa 1080

agtgaaaaag ttttgcacca gactgttgag aatggtaaag taaagttgat tattctctac 1140

caagttatag aagatatcgt tcaaaccaca cctattgtca gggagactga agaatga 1197

<210> 3
<211> 1923
<212> DNA
<213> Bacillus subtilis

<400> 3
gtggctatgg aacagcagca aaacagttat gatgaaaatc agatacaggt actagaagga 60

ttggaagctg ttcgtaaaag accggggatg tatatcggtt cgacaaacag caaaggcctt 120

caccacctgg tatgggaaat tgtcgacaat agtattgacg aagccctcgc cggttattgt 180

acggatatca atatccaaat cgaaaaagac aacagtatca cggttgtaga taatggccgc 240

ggtattccag tcggtattca tgaaaaaatg ggccgtcctg cggtagaagt cattatgacg 300

gtacttcatg ccggaggaaa atttgacgga agcggctata aagtatccgg aggattacac 360

ggtgtaggtg cgtctgtcgt aaacgcacta tcaacagagc ttgatgtgac ggttcaccgt 420

gacggtaaaa ttcaccgcca aacttataaa cgcggagttc cggttacaga ccttgaaatc 480

attggcgaaa cggatcatac aggaacgacg acacattttg tcccggaccc tgaaattttc 540

tcagaaacaa ccgagtatga ttatgatctg cttgccaacc gcgtacgtga attagccttt 600

ttaacaaagg gcgtaaacat cacgattgag gataaacgtg aaggacaaga gcgcaaaaat 660

gaataccatt acgaaggcgg aattaaaagt tatgtagagt atttaaaccg ctctaaagag 720

gttgtccatg aagagccgat ttacattgaa ggcgaaaagg acggcattac ggttgaagtg 780

gctttgcaat acaatgacag ctacacaagc aacatttact cgtttacaaa caacattaac 840

acgtacgaag gcggtaccca tgaagctggc ttcaaaacgg gcctgactcg tgttatcaac 900

gattacgcca gaaaaaaagg gcttattaaa gaaaatgatc caaacctaag cggagatgac 960

gtaagggaag ggctgacagc gattatttca atcaaacacc ctgatccgca gtttgagggc 1020

caaacgaaaa caaagctggg caactcagaa gcacggacga tcaccgatac gttattttct 1080

acggcgatgg aaacatttat gctggaaaat ccagatgcag ccaaaaaaat tgtcgataaa 1140

ggcttaatgg cggcaagagc aagaatggct gcgaaaaaag cccgtgaact aacacgtcgt 1200

aagagtgctt tggaaatttc aaacctgccc ggtaagttag cggactgctc ttcaaaagat 1260

ccgagcatct ccgagttata tatcgtagag ggtgactctg ccggaggatc tgctaaacaa 1320

ggacgcgaca gacatttcca agccattttg ccgcttagag gtaaaatcct aaacgttgaa 1380

aaggccagac tggataaaat cctttctaac aacgaagttc gctctatgat cacagcgctc 1440

ggcacaggta ttggggaaga cttcaacctt gagaaagccc gttaccacaa agttgtcatt 1500

atgacagatg ccgatgttga cggcgcgcac atcagaacac tgctgttaac gttcttttac 1560

agatatatgc gccaaattat cgagaatggc tacgtgtaca ttgcgcagcc gccgctctac 1620

aaggttcaac aggggaaacg cgttgaatat gcgtacaatg acaaggagct tgaagagctg 1680

ttaaaaactc ttcctcaaac ccctaagcct ggactgcagc gttacaaagg tcttggtgaa 1740

atgaatgcca cccagctatg ggagacaacc atggatccta gctccagaac acttcttcag 1800

gtaactcttg aagatgcaat ggatgcggac gagacttttg aaatgcttat gggcgacaag 1860

gtagaaccgc gccgaaactt catagaagcg aatgcgagat acgttaaaaa tcttgacatc 1920

taa 1923

<210>  4
<211>  3582
<212>  DNA
<213>  Bacillus subtilis

<400>  4
ttgacaggtc aactagttca gtatggacga caccgccagc gcagaagcta tgctcgcatt      60

agcgaagtgt tagaattacc aaatctcatt gaaattcaaa cctcttctta tcagtggttt     120

cttgatgagg gtcttagaga gatgtttcaa gacatatcac caattgagga tttcactggt     180

aacctctctc ttgagttcat tgattatagt ttaggtgagc ctaaatatcc tgtagaggaa     240

tcaaaagaac gtgatgtgac ttactcagct ccgctaagag tgaaggttcg tttaattaac     300

aaagaaactg gagaggtaaa agaccaagat gtcttcatgg gtgatttccc tattatgaca     360

gatacaggta cttttatcat taacggtgcg gaacgcgtta tcgtttccca gcttgttcgg     420

tctccaagtg tatatttcag tggtaaagta gacaaaaacg gtaaaaaagg ttttaccgca     480

actgtcattc caaaccgtgg cgcatggtta gaatacgaaa ctgatgcgaa agatgttgtt     540

tatgtccgca ttgatcgcac acgtaagttg ccggttacgg ttcttttgcg tgctctcggc     600

ttcggctccg atcaagagat tcttgatctc ataggagaaa acgaatacct gcgaaatacg     660

cttgataaag ataacacaga aaacagcgac aaagcgttgc tggaaattta cgagcgtctc     720

cgtcctggag agccgcctac agtagaaaat gcgaaaagct tgcttgattc tcgtttcttt     780

gatccgaaac gatacgatct tgccaatgta ggacgctata aaattaataa aaaacttcat     840

attaagaatc gcctcttcaa tcagagactt gctgaaacgc ttgttgatcc tgaaacagga     900

gaaatccttg ctgaaaaagg ccagattctt gatagaagaa cacttgataa agtactgcca     960

tacttagaaa acggaatcgg ttttagaaag ctgtatccga atggcggcgt tgttgaagat    1020

gaagtgactc ttcaatcaat taaaatcttt gctccgactg atcaagaagg agaacaggtt    1080

attaatgtaa tcggcaatgc ttacatcgaa gaagagatta aaaacatcac gcctgctgat    1140

attatttctt caatcagcta cttcttcaac ctgctgcacg gagtaggcga cacagatgat    1200

atcgatcatc ttggaaaccg ccgtttacgt tctgtaggcg agcttctcca gaaccaattc    1260

cgtatcggtt taagccgtat ggagcgtgtg gttcgtgaga gaatgtcaat tcaagatacg    1320

aatacaatta cgcctcagca gctgatcaat attcgtcctg ttattgcgtc cattaaagag    1380

ttctttggaa gctcacagct ttctcaattc atggatcaga cgaacccgct tgctgaatta    1440

acgcacaagc gtcgtctgtc agcattagga ccgggcggat tgacacgtga gcgtgccgga    1500

atggaagtgc gtgacgttca ctactcccac tatggccgta tgtgtccgat tgaaacgcct    1560

gagggcccga acatcggttt gatcaactca ctatcatctt atgcaaaagt aaaccgtttt    1620

ggctttattg aaacgccata tcgccgcgtt gatcctgaaa cagggaaggt aacgggcaga    1680

atcgattact taactgctga tgaagaggat aactatgttg tcgctcaagc gaatgctcgt 1740

cttgatgacg aaggcgcctt tattgatgac agcatcgtag ctcgtttccg cggggagaac 1800

actgttgttt ccagaaatcg tgtagactac atggatgtat cgcctaagca ggttgtatct 1860

gctgcgacag catgtatccc gttcttagaa aacgatgact ccaaccgtgc cctcatggga 1920

gcgaacatgc agcgtcaggc tgtgccttta atgcagccgg aagcgccatt tgttggaact 1980

ggtatggaat acgtatcagg aaaagactct ggtgccgctg ttatttgtaa acaccctggt 2040

atcgttgaac gcgtagaagc gaaaaacgtt tgggttcgcc gttatgaaga agtagacggt 2100

caaaaagtaa aaggaaacct ggataaatac agcctgctga aatttgtccg ctctaaccaa 2160

ggtacgtgct acaaccagcg tccgatcgta agtgtcggcg atgaagtggt aaaaggagaa 2220

atccttgctg acggtccttc tatggagctt ggtgaacttg cacttggccg taacgtaatg 2280

gtcggcttca tgacatggga tggctacaac tatgaggatg ccatcatcat gagtgaacgc 2340

ctagtgaagg atgatgttta tacatctatc cacattgaag aatacgaatc agaagcacgt 2400

gatacgaaac ttggacctga agaaatcact cgcgatattc caaatgtcgg tgaagatgcg 2460

cttcgcaatc ttgatgaccg tggaatcatc cgtattgggg cagaagtaaa agacggagat 2520

cttcttgttg gtaaagtaac gcctaaaggc gtaactgaac tgactgcaga agaacgcctt 2580

cttcacgcca tttttggcga gaaagcccgc gaggttcgtg atacttctct tcgtgtgcct 2640

cacggcggcg gcggaattat ccatgacgtt aaagtcttca accgtgaaga cggagacgaa 2700

cttcctccag gtgttaacca gttagtacgc gtatatatcg ttcagaaacg taagatttct 2760

gaaggggata aaatggccgg tcgtcacggt aacaaaggtg ttatctctaa gattcttcct 2820

gaagaggata tgccttacct tcctgacggc acaccaattg atatcatgct taacccgctg 2880

ggcgtaccat cacgtatgaa catcgggcag gtattggaac ttcacatggg tatggccgct 2940

cgttaccttg gcattcacat tgcatctcct gtatttgacg gagcgcgaga agaggatgtc 3000

tgggaaacac ttgaagaagc cggcatgtct cgtgacgcca aaacagtgct ttacgacgga 3060

cgtactggag agccgtttga taaccgtgta tctgtcggta tcatgtacat gatcaaactg 3120

gctcacatgg ttgacgataa acttcatgca cgctctacag gtccttactc acttgttacg 3180

cagcagcctc ttggcggtaa agcgcaattt ggcggacagc gttttggtga gatggaggtt 3240

tgggcacttg aagcttacgg tgcggcttat actcttcaag aaattctgac tgttaagtct 3300

gatgacgtgg ttggacgtgt gaaaacatac gaagccatcg ttaaaggcga caatgttcct 3360

gaaccaggtg ttccggaatc attcaaagta ttaatcaaag aacttcaaag cttaggtatg 3420

gatgtcaaaa tcctttctgg tgatgaagaa gaaatagaaa tgagagattt agaagacgaa 3480

gaagatgcga aacaagctga cggcctggca ttatcaggtg atgaagagcc ggaagaaaca 3540

gcatctgcag acgttgaacg cgatgtagta acaaaagaat aa 3582

<210> 5
<211> 1632
<212> DNA
<213> Bacillus subtilis

<400> 5
atggcaaaag aaattaagtt tagtgaagaa gctcgccgcg caatgcttcg cggtgtcgat 60

gcacttgctg atgctgttaa agtaacttta ggaccaaaag gacgcaacgt ggttctagag 120

aaaaaattcg gttctccgtt aatcacaaat gacggtgtaa caattgctaa agagatcgag 180

ctagaagacg cgtttgaaaa catgggtgct aagcttgttg ctgaagtagc cagcaaaaca 240

aacgacgttg ccggtgacgg tacaacaact gcgacagttc ttgcgcaagc aatgatccgt 300

gaaggcctta aaaacgtaac agcaggcgct aaccctgtag gcgtgcgtaa aggtatggaa 360

caagctgtag cggttgcgat cgaaaactta aaagaaattt ctaagccaat cgaaggcaaa 420

gagtctatcg ctcaggttgc tgcgatctct gcggctgatg aggaagtcgg aagccttatc 480

gctgaagcaa tggagcgcgt aggaaacgac ggcgttatca caatcgaaga gtctaaaggc 540

ttcacaactg agcttgaagt tgttgaaggt atgcaattcg accgcggata tgcgtctcct 600

tacatggtaa ctgactctga taagatggaa gcggttcttg acaatcctta catcttaatc 660

acagacaaaa aaatcacaaa cattcaagaa atccttcctg tgcttgagca ggttgttcag 720

caaggcaaac cattgcttct gatcgcagaa gacgttgaag gcgaagcact tgcaacactt 780

gttgtgaaca aacttcgcgg cacattcaac gcagttgctg ttaaagctcc tggcttcggt 840

gaccgccgta aagcaatgct tgaagacatc gctgtcctta ctggcggaga agtcatcaca 900

gaagatcttg gccttgacct gaaatctact caaatcgctc aattgggacg cgcttctaaa 960

gttgtcgtta ctaaagaaaa cacaacaatc gttgaaggcg ctggcgaaac agacaaaatt 1020

tctgcccgcg tgactcaaat ccgcgctcaa gtggaagaaa caacttctga attcgacaga 1080

gaaaaattac aagagcgtct tgctaaactt gctggcggcg tagctgtcat caaagtcggc 1140

gctgcgactg aaactgaact gaaagagcgt aaacttcgca tcgaagacgc cctgaactca 1200

actcgcgcag ctgttgaaga aggcatcgta tccggtggtg gtacagcgct tgtaaacgta 1260

tataacaaag tcgctgcagt tgaagctgaa ggcgatgctc aaacaggtat caacatcgtg 1320

cttcgcgcgc ttgaagagcc aatccgtcaa atcgcacata acgctggtct tgaaggatct 1380

gtcatcgttg agcgcctcaa aaacgaagaa atcggcgtag gcttcaacgc tgcaactggc 1440

gaatgggtaa acatgatcga aaaaggtatc gttgacccaa cgaaagttac acgctcagct 1500

cttcaaaacg ctgcgtctgt agctgcaatg ttcttaacaa ctgaagccgt tgtcgctgac 1560

aagccagaag aaaacgctgg cggcggaatg cctgacatgg gcggcatggg cggtatgggc 1620

ggcatgatgt aa 1632